# EUROPEAN PATENT APPLICATION

(11) **EP 1 621 528 A1**
(43) Date of publication of application: **01.02.2006**
(21) Application number: 04729720.5
(22) Date of filing: 27.04.2004
(51) Int. Cl.: C07C 201/08, C07C 205/59

(54) **PROCESS FOR PRODUCING 3-NITRO-4-ALKOXYBENZOIC ACID**

(30) Priority: 02.05.2003 JP 2003127030
(71) Applicant: Kabushiki Kaisha Ueno Seiyaku Oyo Kenkyujo, Osaka-shi, Osaka 541-0043 (JP)
(72) Inventor: UENO, Ryuzo, Nishinomiya-shi, Hyogo 662-0038 (JP); KITAYAMA, Masaya, Takarazuka-shi, Hyogo 665-0881 (JP); WAKAMORI, Hiroyuki, Tanba-shi, Hyogo 669-3105 (JP)
(74) Representative: Albrecht, Thomas
(86) International application number: PCT/JP2004/006055
(87) International publication number: WO 2004/096750

(57) **Abstract**

The present invention provides a process for preparing 3-nitro-4-alkoxybenzoic acid, which comprises the step of subjecting a mixture comprising 4-alkoxybenzoic acid and 40-80% nitric acid, wherein the amount of the 40-80% nitric acid is equal to or more than 8 times that of 4-alkoxybenzoic acid by weight, to a reaction at a temperature of 30-100°C.

## Description

### TECHNICAL FIELD

The present invention relates to a process for preparing 3-nitro-4-alkoxybenzoic acid by selective nitration of 4-alkoxybenzoic acid at position 3.

### BACKGROUND ART

3-Nitro-4-alkoxybenzoic acid is an important compound as an intermediate for preparing products such as dyes, pigments, and medicines. Particularly, 3-nitro-4-methoxybenzoic acid (referred to as 3-nitroanisic acid hereinafter) is an important compound as an intermediate for preparing Red KD Base(DB-40), Red KL Base(DB-20) or the like which is widely used as a diazo component to synthesize an azo dye.

Generally, nitration of an aromatic compound such as 4-alkoxybenzoic acid is performed by using a mixed acid of concentrated nitric acid and concentrated sulfuric acid. The conventional nitration reaction using the mixed acid, however, generates side products such as dinitro compounds depending on the reaction condition, and therefore, it is difficult to be conducted in commercial scale. Further, such a method using the mixed acid generates a lot of wasted sulfuric acid which may affect the environment.

A conventional method for preparing 3-nitro-4-alkoxybenzoic acid via nitration of 4-alkoxybenzoic acid at position 3 is, for example, that in which the nitration is performed by using 60% nitric acid as a nitrating agent in a halogenated hydrocarbon solvent such as carbon tetrachloride which is inactive against the nitration reaction in the presence of a catalytic amount of sulfuric acid (Kameo takashi and Hirashima Tsuneaki, "Nitration of several aromatic compounds with nitric acid in an inactive solvent", KAGAKU TO KOGYO, Vol.59, 2, 49-53, 1985).

This method has a problem of environmental impact arising from the use of a halogenated hydrocarbon solvent. In addition, recycling of the reaction product (i.e. collection of nitric acid used as a nitrating agent) is difficult because the nitric acid is mixed with sulfuric acid. There is another problem regarding to generation of a lot of acidic waste solution.

Another conventional method is that comprising adding nitric acid by drops to a mixture of sulfuric acid and 4-alkoxybenzoic acid and causing a nitration reaction with cooling at a temperature equal to or less than 10°C (JP S 55-31045A).

This method comprises the steps of carrying out a nitration reaction, mixing the reaction product with a large amount of cold water to precipitate 3-nitro-4-methoxybenzoic acid, and collecting the precipitates. The method, therefore,- has a problem of generation of a lot of diluted acidic waste solution containing nitric acid and sulfuric acid. It is difficult to collect nitric acid used as the nitrating agent from such a diluted acidic waste solution to recycle the nitric acid.

Furthermore, the nitration reaction requires a long time because it is carried out at a low temperature so that side reactions including dinitration are suppressed. In addition, the method requires a huge amount of energy to cool the refrigerant when it is employed for commercial production.

### DISCLOSURE OF THE INVENTION

An object of the present invention is to provide a process for preparing 3-nitro-4-alkoxybenzoic acid in high yield, without the production of a lot of acidic waste solution containing sulfuric acid and nitric acid, the use of a solvent like halogenated hydrocarbon, and the production of side products such as dinitro compounds.

The present invention provides a process for preparing 3-nitro-4-alkoxybenzoic acid, which comprises the step of subjecting a mixture comprising 4-alkoxybenzoic acid and 40-80% nitric acid, wherein the amount of the 40-80% nitric acid is equal to or more than 8 times that of 4-alkoxybenzoic acid by weight, to a reaction at a temperature of 30-100°C.

The present invention also provides the process for preparing 3-nitro-4-alkoxybenzoic acid as described above, which further comprises the steps of cooling the reaction product resulting from the above process, and isolating and collecting 3-nitro-4-alkoxybenzoic acid precipitated thereby.

The present invention further provides the process for preparing 3-nitro-4-alkoxybenzoic acid as described above, which further comprises the steps of heating the reaction product resulting from the above process to give a homogenous solution, cooling the solution, and isolating and collecting 3-nitro-4-alkoxybenzoic acid precipitated thereby.

Furthermore, the present invention provides the process for preparing 3-nitro-4-alkoxybenzoic acid as described above, which further comprises the steps of cooling the reaction product resulting from the above process, isolating 3-nitro-4-alkoxybenzoic acid precipitated thereby from the liquid phase and collecting the liquid phase, and adjusting the concentration of nitric acid in the liquid phase to 40-80% so that the liquid phase is used again for the reaction step.

The process of the present invention allows preparation of 3-nitro-4-alkoxybenzoic acid of high purity in high yield without the use of a halogenated hydrocarbon solvent and the production of acidic waste solution containing sulfuric acid and nitric acid.

Further, the process of the present invention accomplishes selective mononitration of 4-alkoxybenzoic acid and generates substantially no, or little, if any, side products such as dinitro compounds

### BEST MODE FOR CARRYING OUT THE INVENTION

In the present specification, "concentration of nitric acid" means concentration of an aqueous nitric acid solution (% by weight).

4-Alkoxybenzoic acid according to the following formula I is preferably used as a starting material for the process of the present invention. Preferably, 4-methoxybenzoic acid (referred to as anisic acid hereinafter), 4-ethoxybenzoic acid, 4-n-propoxybenzoic acid, 4-isopropyloxybenzoic acid, or 4-n-butoxybenzoic acid, more preferably anisic acid, may be used. 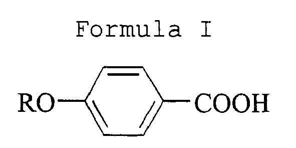
wherein R is a straight or branched, saturated alkyl group with a carbon number of 1 to 6, optionally having a substituted group. Examples of the substituted group may include halogen atoms and hydroxyl, methoxy, ethoxy, carboxy, methoxycarbonyl, ethoxycarbonyl, cyano, nitro, amino, carbonyl and thiocarbonyl groups.

The concentration of nitric acid to be used in the present invention is preferably 40-80%, and more preferably 50-60%. When the concentration is higher than 80%, side products will increase. At the same time, when it is lower than 40%, the reaction will take a long time, and the starting material may contaminate the objective compound because the conversion rate will be decreased.

The amount of nitric acid to be used in the process of the present invention is preferably equal to or more than 8 times, more preferably 10-20 times, that of 4-alkoxybenzoic acid by weight. If it is less than 8 times by weight, stirring the mixture to make it homogenous is difficult and the reaction time becomes longer. In addition, problems including an increase of side products and a decrease of the yield may arise. Although the amount of nitric acid to be used may be more than 20 times that of 4-alkoxybenzoic acid by weight, such an amount does not bring any superior effect compared to the amount less than 20 times by weight. Instead, it results in high production cost in view of the use efficiency of the reactor.

In the process of the present invention, the reaction temperature for nitration is preferably 30-100°C, and more preferably 60-95°C. Selective mono-nitration efficiently proceeds in the temperature range. When the reaction temperature is less than 30°C, the reaction rate becomes low and then the production efficiency will be decreased. When the reaction temperature is higher than 100°C, several problems such as an increase of side products and bubbling of the reaction product induced by decarboxylation will arise.

The reaction time may vary depending on the concentration of nitric acid, the ratio of the amount of nitric acid to that of the starting material, and the reaction temperature, and is preferably 0.2-2 hours.

The process of the present invention may be conducted under any condition of pressure. In general, it is preferably conducted under atmospheric pressure.

3-Nitro-4-alkoxybenzoic acid produced as described above is deposited as crystalline product (i, e, precipitated) after the nitration reaction by cooling the reaction product. Precipitation of 3-nitro-4-alkoxybenzoic acid from the reaction product may be conducted by cooling the suspension resulting from the reaction. Alternatively, the reaction product (i. e., the suspension) may be heated to give a homogenous solution and then cooled to precipitate the desired product. In the latter case, the suspension may be heated to, but not limited to, 80 to 90°C. According to the method, 3-nitro-4-alkoxybenzoic acid of higher quality can be obtained due to the purifying effect of re-precipitation.

3-Nitro-4-alkoxybenzoic acid precipitated by cooling the reaction product is isolated and collected from the liquid phase by means of conventional methods such as centrifugation and filter press.

The liquid phase obtained after the isolation can be used again in the reaction step. In this embodiment, the concentration of nitric acid in the liquid phase is adjusted in advance to the range preferably used in the process of the present invention by concentrating the same or adding a high concentration of nitric acid.

The process of the present invention does not involve sulfuric acid and organic solvents, and there is no need for isolation and purification of nitric acid from the liquid phase. The recycling of nitric acid, therefore, can be carried out efficiently.

3-Nitro-4-alkoxybenzoic acid of high quality can be isolated from the liquid phase in high yield. After washed with water and dried, the 3-nitro-4-alkoxybenzoic acid can be processed to provide a commercial product which is preferably used as an intermediate for manufacturing products such as dyes, pigments, and medicines.

The process of the present invention can be conducted in both of batch-wise and sequential manners.

### INDUSTRIAL APPLICABILITY

3-Nitro-4-alkoxybenzoic acid obtained by the process of the present invention can be preferably used as an intermediate for manufacturing products such as dyes, pigments, and medicines.

### EXAMPLE

The present invention is described in details by the following Examples and Comparative Examples.

The purity of 3-nitro-4-alkoxybenzoic acid obtained in each Example or Comparative Example was analyzed by high-performance liquid chromatography.

### Example 1

Preparation of 3-nitroanisic acid by the process of the present invention

Ten grams of anisic acid was suspended into 140 g of 60 % nitric acid and the suspension was heated to 90°C over 30 min such that the anisic acid was completely dissolved. The solution thus obtained was stirred for 30 min at 90°C in order that the nitration reaction completed. The reaction product which is in a solution state was gradually cooled to room temperature to give crystalline product. The crystalline product was collected by filtration, and the product thus collected was well washed with water and dried. As a result, 11.5 g of white crystalline product was obtained (the yield was 88.5%). The resulting crystalline product was 3-nitroanisic acid of which purity was 99.6%.

The process of the present invention could provide 3-nitroanisic acid of high purity in high yield.

### Example 2

Recycling of the liquid phase left in Example 1 for the process of the present invention

The liquid phase obtained after the filtration and the collection of 3-nitroanisic acid in Example 1 was added with an appropriate amount of 98% nitric acid such that the concentration of nitric acid of the liquid phase became 60%. To 141.1 g of the liquid phase of which concentration of nitric acid was thus adjusted, 8.9g of anisic acid was suspended. In a similar way to Example 1, 11.6 g of white crystalline product was obtained. The yield of 3-nitroanisic acid calculated from the amount of the added anisic acid was 99.4%. This means that the liquid phase obtained after the reaction could be recycled through adjustment of the concentration, with the yield of 3-nitroanisic acid of substantially nearly 100%.

Furthermore, the purity of the crystalline product of 3-nitroanisic acid thus obtained was 99.5%.

The process of the present invention, which comprises recycling of the liquid phase obtained after the preceding reaction, could provide 3-nitroanisic acid in an efficient manner.

### Example 3

Preparation of 4-ethoxy-3-nitrobenzoic acid by the process of the present invention

In a similar way to Example 1, except for using 10.9 g of 4-ethoxybenzoic acid instead of the anisic acid used in Example 1, 12.2 g of white crystalline product was obtained (the yield was 87.8%). The purity of the crystalline product of 4-ethoxy-3-nitrobenzoic acid thus obtained was 99.4%.

### Example 4

### Preparation of 4-n-propoxy-3-nitrobenzoic acid by the process of the present invention

In a similar way to Example 1, except for using 11.8 g of 4-n-propoxybenzoic acid instead of the anisic acid used in Example 1, 11.2 g of white crystalline _product was obtained (the yield was 75.7%). The purity of the crystalline product of 4-n-propoxy-3-nitrobenzoic acid thus obtained was 99.2%.

### Example 5

Preparation of 4-isopropyloxy-3-nitrobenzoic acid by the process of the present invention

In a similar way to Example 1, except for using 11.8 g of 4-isopropyloxybenzoic acid instead of the anisic acid used in Example 1, 11.8 g of white crystalline product was obtained (the yield was 79.8%). The purity of the crystalline product of 4-isopropyloxy-3-nitrobenzoic acid thus obtained was 99.4%.

### Example 6

Preparation of 4-n-butoxy-3-nitrobenzoic acid by the process of the present invention

In a similar way to Example 1, except for using 12.7 g of 4-n-butoxybenzoic acid instead of the anisic acid used in Example 1, 11.9 g of white crystalline product was obtained (the yield was 76.2%). The purity of the crystalline product of 4-n-butoxy-3-nitrobenzoic acid thus obtained was 99.2%.

### Comparative Example 1

### Preparation using a low concentration of nitric acid

In a similar way to Example 1, except for using 200 g of 30% nitric acid instead of the nitric acid used in Example 1, and stirring at 90°C for 5 hours instead of 30 min, 7.8 g of white crystalline product was obtained. The purity of the crystalline product regarding to 3-nitroanisic acid was 90%, and the crystalline product contained 10% of anisic acid as an impurity which was the unreacted starting material.

When the concentration of nitric acid was low, the purity of the resulting crystalline product and the reaction efficiency were low compared to those achieved by the process of the present invention.

### Comparative Example 2

### Preparation using a high concentration of nitric acid

In a similar way to Example 1, except for using 140 g of 98% nitric acid instead of the nitric acid used in Example 1, and carrying out a nitration reaction at 80°C, 7.2 g of white crystalline product was obtained. The crystalline product thus obtained, however, did not contain the objective 3-nitroanisic acid and mainly consisted of compounds formed by decarboxylation such as 2, 4-dinitroanisole and 2, 4-dinitrophenol.

When the concentration of nitric acid was high, the objective product, which was expected to be formed by the selective mononitration, was not obtained.

### Comparative Example 3

Preparation in which a nitration reaction was carried out using a mixed acid at room temperature

Ten grams of anisic acid was suspended into 45 g of 98% sulfuric acid and the suspension was cooled on ice. To the suspension, a mixed acid consisting of 8.4 g of 60% nitric acid and 8.0 g of 98% nitric acid was added by drops over 20 min. After the addition, the suspension was stirred for 1 hour at room temperature such that the nitration reaction completed. The reaction product was put into 200 g of ice-cold water, and the crystalline product precipitated thereby was collected by filtration. The crystalline product was well washed and dried, and 11.9 g of flesh-colored crystalline product was obtained. The purity of the crystalline product regarding to 3-nitroanisic acid was 70%, and the crystalline product contained impurities such as 2, 4-dinitroanisole.

The resulting crystalline product showed a low purity, and the liquid phase obtained after the filtration could not be recycled.

### Comparative Example 4

Preparation in which a nitration reaction was carried out with a mixed acid with cooling

Ten grams of anisic acid was dissoleved in 200 g of 98% sulfuric acid to give a solution and the solution was cooled to -5°C or less. To the solution, a mixed acid consisting of 6.6 g of 60% nitric acid and 50 g of 98% sulfuric acid was added by drops over about 45 min. During the addition, the temperature of the reaction product was watched so as not to be more than 0°C due to the heat generation. After the addition, the solution was stirred for 1 hour at about -5°C in order that the nitration reaction completed. The reaction product was put into 500 g of ice, and the crystalline product precipitated thereby was collected by filtration. The crystalline product was well washed with water and then dried. As a result, 12.6 g of white crystalline product was obtained. The purity of the crystalline product thus obtained in relation to 3-nitroanisic acid was 95%.

The resulting crystalline product showed such a low purity and the liquid phase obtained after the filtration could not be recycled.

## Claims

1. A process for preparing 3-nitro-4-alkoxybenzoic acid, which comprises the step of subjecting a mixture comprising 4-alkoxybenzoic acid and 40-80% nitric acid, wherein the amount of the 40-80% nitric acid is equal to or more than 8 times that of 4-alkoxybenzoic acid by weight, to a reaction at a temperature of 30-100°C.

2. The process of Claim 1, which further comprises the steps of cooling the reaction product, and isolating and collecting 3-nitro-4-alkoxybenzoic acid precipitated thereby.

3. The process of Claim 1, which further comprises the steps of heating the reaction product to give a homogenous solution, cooling the solution, and isolating and collecting 3-nitro-4-alkoxybenzoic acid precipitated thereby.

4. The process of Claim 1, which further comprises the steps of cooling the reaction product, isolating 3-nitro-4-alkoxybenzoic acid precipitated thereby from the liquid phase and collecting the liquid phase, adjusting the concentration of nitric acid in the liquid phase to 40-80%, and using thus obtained liquid phase for the reaction step.

5. The process of any one of Claims 1 to 4, wherein 4-alkoxybenzoic acid is selected from the group consisting of 4-methoxybenzoic acid, 4-ethoxybenzoic acid, 4-n-propoxybenzoic acid, 4-isopropyloxybenzoic acid, and 4-n-butoxybenzoic acid.
